(19) Europäisches Patentamt
European Patent Office
Office européen des brevets

(11) Veröffentlichungsnummer: **0 192 608 B1**

(12) **EUROPÄISCHE PATENTSCHRIFT**

(45) Veröffentlichungstag der Patentschrift: **24.07.91 Patentblatt 91/30**

(51) Int. Cl.$^5$: **C07C 31/30**, C07C 29/70

(21) Anmeldenummer: **86810081.9**

(22) Anmeldetag: **13.02.86**

(54) **Verfahren zur Herstellung von Alkalialkoholaten.**

(30) Priorität: **20.02.85 CH 791/85**

(43) Veröffentlichungstag der Anmeldung: **27.08.86 Patentblatt 86/35**

(45) Bekanntmachung des Hinweises auf die Patenterteilung: **24.07.91 Patentblatt 91/30**

(84) Benannte Vertragsstaaten: **CH DE FR GB IT LI**

(56) Entgegenhaltungen:
**DE-A- 2 612 642**
**FR-A- 1 070 601**
**FR-A- 2 235 901**
**GB-A- 629 184**

(73) Patentinhaber: **CIBA-GEIGY AG**
**Klybeckstrasse 141**
**CH-4002 Basel (CH)**

(72) Erfinder: **Surber, Werner, Dr.**
**Vorderbergstrasse 76**
**CH-4104 Oberwil (CH)**

**EP 0 192 608 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Alkalialkoholate tertiärer Alkohole haben in letzter Zeit als Katalysatoren und Konditioniermittel wachsendes Interesse gefunden, da die entsprechenden sterisch stark gehinderten Alkohole infolge ihrer sehr geringen Acidität besonders starke Protonen-Akzeptoren sind und meist keine Nebenreaktion eingehen. Insbesondere bei der Herstellung von Diketo-pyrrolopyrrol-Pigmenten, wie sie beispielsweise die EP-A 94911 beschreibt, sind Alkali-tertiär-Alkoholate wichtig.

Aus der GB-A-629 184 ist ein Verfahren zur Herstellung primärer Alkali-Alkoholate, bei dem man einen primären aliphatischen Alkohol mit bis zu 4 C-Atomen zu geschmolzenem Alkali zugibt, bekannt. Es ist aber auch bekannt, und in der DE-A-2 612 642 bestätigt, dass tertiäre Alkohole viel träger reagieren als primäre und dass die Reaktivität der Alkohole mit verzweigter Kette gegenüber Alkalimetalle so gering sei, dass für die Herstellung von Alkoholaten nach den bislang bekannten Verfahren (ohne Zusatz eines inerten Lösungsmittels) technisch unbrauchbar lange Reaktionszeiten erforderlich waren.

Bisher erfolgte die Herstellung der Alkali-tert.-Alkoholate im technischen Masstab in einem inerten Lösungsmittel wie z.B. Cyclohexan (siehe z.B. DE-OS 2,612,642). Dies hat jedoch den Nachteil, dass das Lösungsmittel von dem bei der Reaktion entstehenden Alkohol wieder abgetrennt werden muss. Auch nach den in der DE-AS 2 333 634 und der GB-PS 746 400 beschriebenen Verfahren zur Herstellung von Alkalimetallalkoholaten werden inerte Lösungsmittel verwendet.

Die vorliegende Erfindung betrifft nun ein Verfahren zur Herstellung von Alkali-tert.-Alkoholaten, wonach der heisse Alkohol dem geschmolzenen Alkalimetall unter Rühren ohne Verwendung eines Lösungsmittels zugegeben wird.

Als Alkalimetalle verwendet man beispielsweise Li oder K, insbesondere aber Na. Das Aufschmelzen des Alkalimetalls erfolgt zweckmässig unter Stickstoff.

Als tert. Alkohole verwendet man vorzugsweise solche mit 5-22 C-Atomen, wie z.B. 3,7-Dimethyl-1,6-octadien-3-ol (Linalool), 3,7,11-Trimethyl-3,6,10-dodocatrien-3-ol, 3,7,11,15-Tetramethyl-1-hexadecen-3-ol, Tetrahydrolinalool oder insbesondere tert. Amylalkohol.

Man verwendet zweckmässig bis zu 5 Mol, bevorzugt 3 bis 4 Mol des Alkohols auf 1 Atomgewicht des Metalls.

Die Zugabe des Alkohols zum geschmolzenen Alkali erfolgt allmählich unter sehr starkem Rühren. Nach der Zugabe des Alkohols wird noch so lange am Rückfluss erhitzt, bis sich alles Alkalimetall gelöst hat. Die so erhaltene alkoholische Lösung kann direkt für Synthesen, insbesondere für die Herstellung von Diketopyrrolopyrrolen eingesetzt werden.

Beispiel 1: In einen trockenen, mit Stickstoff gefüllten 630 lt-Rührkessel, werden 10,4 kg Na-Stangen zugegeben. Durch Erhöhen der Manteltemperatur auf 100-105°C wird das Na geschmolzen.

In einen trockenen, mit Stickstoff gefüllten 250 lt-Rührkessel mit Ankerrührer und Rückflusskühler, werden 150 kg t-Amyl-alkohol eingesogen und zum Sieden erhitzt. Dieser kochend heisse Amylalkohol wird mit trockenem Stickstoff langsam zum geschmolzenen Na unter Rühren gedrückt. Unter sehr starkem Rühren und Heizen mit Manteldampf wird bei einer Innentemperatur von 102-114°C während 4 Stunden gekocht, bis kein Wasserstoff mehr gebildet wird, d.h. alles Na in Lösung gegangen ist. Man erhält eine klare, farblose bis gelbliche Lösung des t-Amylalkoholates, welche direkt für Synthese eingesetzt werden kann.

Beispiel 2: Zu 48,2 l der gemäss Beispiel 1 erhaltenen Lösung enthaltend 17,3 kg Na.tert.amylat werden bei 98°C 7,22 kg Benzonitril, dann eine möglichst wasserfreie Lösung von 7,31 kg Bernsteinsäure-dimethylester in 5,0 l tert.-Amylalkohol innert 145 Minuten mit einer Dosierpumpe zugefügt. Die Temperatur wird stets bei 98-99°C gehalten. Das entstehende Methanol destilliert ab. Am Ende der Zudosierung wird das Reaktionsgemisch noch 2 Stunden bei 99°C gehalten. Man kühlt anschliessend auf 65°C, verdünnt langsam mit 100 l Methanol, neutralisiert langsam mit 10,8 l Eisessig und kocht kurz bei Rückflusstemperatur. Die erhaltene Pigment-Suspension wird bei ca. 50°C filtriert. Der Filterkuchen wird in 300 l Methanol aufgeschlämmt, und das Pigment wird wieder filtriert. Schliesslich wäscht man es farblos mit Methanol und Wasser und trocknet es bei 80°C im Vakuum. Man erhält 9,04 kg (entsprechend 62,8 % d.Th., bezogen auf Bernsteinsäure-dimethylester) reines Pigment der Formel

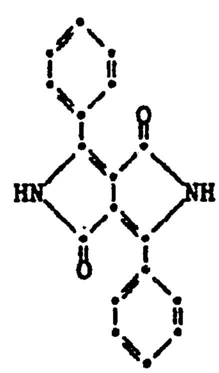

,

das, in Weich-PVC in 0,2 %-iger Konzentration eingearbeitet, eine rote Färbung ergibt.

Beispiel 3: In einem trockenen mit Stickstoff gefüllten 750 ml-Sulfierkolben mit Flügelrührer und Rückflusskühler werden 10,4 g Na geschmolzen, indem das Oelbad des Reaktionskolbens auf 120°C

geheizt wird. Nun werden 260 g 3,7-Dimethyl-1,6-octadien-3-ol (Linalool) von 100°C durch einen Tropftrichter unter Rühren innert 30 Min. zum geschmolzenen Na gegeben. Schliesslich wird die Innentemperatur auf 115-120°C erhöht. Nach ca. 90 Min. entsteht kein Wasserstoff mehr und alles Na ist in Lösung gegangen. Es entsteht eine gelbe Lösung.

Beispiel 4 : Anstelle von 260 g Linalool können 370,7 g 3,7,11-Trimethyl-3,6,10-dodecatrien-3-ol verwendet werden. Die Durchführung ist dieselbe wie im Bsp. 3 hingegen beträgt die Reaktionsdauer bis zur vollständigen Umsetzung des Na ca. 5 Std. Es entsteht eine hellbraune Lösung.

Beispiel 5 : 500 g 3,7,11,15-Tetramethyl-1-hexadecen-3-ol von 100°C werden in 15 Min. unter Rühren zu 10,4 g Na geschmolzen bei 108-109°C zulaufen gelassen bei einer Oelbadtemperatur von 125°C. Nach 5 Std. Rühren bei einer Innentemperatur von 109-130°C ist die $H_2$-Entwicklung bzw. die Reaktion beendet. Es entsteht eine gelbe Lösung.

Beispiel 6 : 264 g Tetrahydrolinalool von 100°C werden zu 10,4 g Na geschmolzen in 30 Min. bei einer Oelbadtemperatur von 125°C zugegeben. Es wird ca. 8 Std. bei einer Innentemperatur von 120°C gerührt bis alles Na in Lösung gegangen ist und kein Wasserstoff-Gas mehr entsteht. Das Na-Salz des t-Akohols ist gelb.

## Patentansprüche

1. Verfahren zur Herstellung von Alkali-tertiär-Alkoholaten durch Umsetzen eines Alkalimetalls mit einem tertiären Alkohol, dadurch gekennzeichnet, dass man den heissen Alkohol zum geschmolzenen Alkalimetall unter Rühren zugibt.

2. Verfahren gemäss Anspruch 1, dadurch gekennzeichnet, dass man als Alkalimetall Natrium verwendet.

3. Verfahren gemäss Anspruch 1, dass man als tertiären Alkohol einen solchen mit 5-22 C-Atomen verwendet.

4. Verfahren gemäss Anspruch 3, dadurch gekennzeichnet, dass man als Alkohol tert. Amylalkohol verwendet.

5. Verfahren gemäss Anspruch 1, dass man bis zu 5 Mol des Alkohols auf ein Atomgewicht des Metalls verwendet.

## Claims

1. A process for the preparation of an alkali metal tertiary alcoholate by reacting an alkali metal with a tertiary alcohol, which process comprises adding the hot alcohol, with stirring, to the melted alkali metal.

2. A process according to claim 1, wherein the alkali metal used is sodium.

3. A process according to claim 1, wherein the tertiary alcohol used is a tertiary alcohol containing 5 to 22 carbon atoms.

4. A process according to claim 3, wherein the alcohol used is tert-amyl alcohol.

5. A process according to claim 1, which comprises the use of up to 5 moles of the alcohol per 1 gram atom of the metal.

## Revendications

1. Procédé pour préparer des alcoolates tertiaires de métaux alcalins par réaction d'un métal alcalin avec un alcool tertiaire, procédé caractérisé en ce qu'on ajoute l'alcool chaud, tout en agitant, au métal alcalin fondu.

2. Procédé selon la revendication 1 caractérisé en ce qu'on utilise le sodium comme métal alcalin.

3. Procédé selon la revendication 1 caractérisé en ce qu'on utilise, comme alcool tertiaire, un alcool de ce genre qui contient de 5 à 22 atomes de carbone.

4. Procédé selon la revendication 3 caractérisé en ce qu'on utilise le tert-pentanol comme alcool.

5. Procédé selon la revendication 1 caractérisé en ce qu'on utilise jusqu'à 5 mol de l'alcool par atome-gramme du métal.